# EUROPEAN PATENT APPLICATION

(11) **EP 4 706 421 A2**
(43) Date of publication of application: **11.03.2026**
(21) Application number: 25200739.8
(22) Date of filing: 08.09.2025
(51) Int. Cl.: A24F 40/40, A61M 21/02, A24F 40/10

(54) **VAPING DEVICE WITH INTEGRAL MANUAL SPINNER**

(30) Priority: 06.09.2024 US 202418826609
(71) Applicant: Ayxter, LLC, Fort Lauderdale, Fl 33309 (US)
(72) Inventor: KAPLAN, Lex, Fort Lauderdale, 33309 (US); GOLDSTEIN, Lindsey, Fort Lauderdale, 33309 (US)
(74) Representative: Schulz Junghans Patentanwälte PartGmbB

(57) **Abstract**

Described is a method and vaping device for relieving anxiety, stress or nervousness. The vaping device (100) is operated to provide vaporized liquid solution to a person and includes a spinner assembly (104) to rotate the vaping device about said the spinner assembly. The device is also used in a method to relieve anxiety, stress or nervousness in a person by actuating the vaping device, inhaling vapor, and actuating the spinning assembly before, after, or between actuations of the vaping device.

## Description

### BACKGROUND OF THE INVENTION

Nicotine is used daily by millions of people for reducing feelings of agitation, anxiety, nervousness, or stress in a person. Nicotine is commonly administered to the bloodstream via inhalation into the lungs by smoking of tobacco (burning of a cigar or cigarette), and more recently be "vaping," i.e., inhaling a vaporized and aerosolized liquid solution containing nicotine.

Vaping devices, also known as electronic cigarettes, or e-cigarettes, are electronic devices that simulate the act of smoking, without the production of potentially harmful by-products that can be formed in the process of burning tobacco or other plant material. Vaping devices heat a liquid solution containing nicotine and vaporize the liquid into an aerosol, which is then inhaled by the user. Vaping devices have gained popularity as an alternative to traditional tobacco smoking.

The components of a typical vaping device can include:
∘ A battery, used as a power source.
∘ A tank or atomizer to hold the liquid solution.
∘ A coil, as a heating element.
∘ A liquid solution, which is vaporized for inhalation.
∘ A wick, to transfer the liquid solution to the coil, and
∘ Airflow Control, as an optional component to customize the mixture of air and vapor.

The above components are typically provided within a single elongate housing unit forming a body of the vaping device. Vaping devices can be fashioned in various shapes, sizes, and materials, including plastic, metal, and even exotic materials like wood or ceramic. They can be customized with different colors, patterns, and even LED lights. A preferred vaping device has a generally elongate body providing a first end comprising the coil for heating the liquid solution and a second end forming a mouthpiece for inhaling vaporized liquid solution. Such vaping devices are commonly cylindrical (round, circular, or oval in cross-section), or cuboid (having a cross-section which is square or rectangular in shape, forming a top face, bottom face, left face and right face). Other shapes, such as conical, or multifaceted (having more than four faces), are also contemplated. The elongate shape of the vaping device can facilitate holding and using the vaping device.

Spinner novelty toys, often simply referred to as fidget spinners, finger spinners, manual spinners, or simply "spinners," are small, hand-held devices designed with a central bearing surrounded by two or three prongs, which can be spun between the fingers, preferably between the index finger or middle finger and the thumb of one hand. As they rotate, they produce a pleasing and often mesmerizing visual effect, and are popularly used for reducing feelings of agitation, anxiety, nervousness, or stress in a person.

Spinners were originally designed as tools to help individuals with attention disorders, such as ADHD and autism, helping to focus and redirect restless energy. Spinners recently gained popularity among young people and students, through social media.

Spinners can also be provided in various shapes, sizes, and materials, including plastic, metal, and even exotic materials like wood or ceramic. Spinners can also be customized with different colors, patterns, and LED lights. The central bearing, usually a ball bearing, facilitates smooth spinning.

Many people find fidget spinners helpful for stress relief and anxiety management. The rhythmic motion and tactile feedback of spinning the toy can have a soothing effect, reducing feelings of stress or restlessness.

What is lacking in the art is a vaping device that includes a feature which can aid in reducing agitation, anxiety, nervousness, or stress in a person between inhalations from the vaping device.

The subject invention advantageously provides a combination vaping device and spinner which can achieve reducing feelings of agitation, anxiety, nervousness, or stress in a person by vaping of nicotine or other substance and can also aid in reducing feelings of agitation, anxiety, nervousness, or stress between vaping occurrences using a spinner device integrated into or integral with the body of the vaping device.

### SUMMARY OF THE INVENTION

The subject invention is directed to a vaping device comprising a spinner and a method for using said device for relieving anxiety, stress or nervousness in a person in need thereof.

The vaping device according to the subject invention comprises a chassis having a heating element and a reservoir for storing a liquid solution that is deliverable to the heating element for heating and vaporization. The chassis further includes a power source for actuating the heating element on and off. Centrally disposed within the chassis is a spinner assembly allowing the vaping device to rotate about the spinner assembly. The vaping device further includes a housing defining a chamber for receiving the chassis. The housing has an aperture that aligns with the spinner assembly such that the spinner assembly can be accessed through the housing with thumb and finger. The housing further includes an inhaling end for inhaling the vaporized liquid solution and a second end.

As will be further appreciated from the description herein, the vaping device can further include a power source that is rechargeable. The rechargeable power source can be in communication with a charging port included on the chassis so that it may be recharged when needed. The power source can be turned on and off by an actuator button disposed in the housing and in operational communication with the heating element. The charging port can be disposed on, and accessible from the second end of the housing. The inhaling end of the housing can include a mouthpiece. The mouthpiece can be a disposable mouthpiece that is separable from the vaping device and replaceable. The vaping device can further include an airflow control. The airflow control can be disposed on and operated from the housing so that the amount of air drawn into the vaping device can be adjusted.

The chassis can further include a wick for delivering liquid solution from the reservoir to the heating element. The spinner assembly can be affixed to the chassis by a positioning ring. The vaping device can further comprise a counterweight disposed within the chamber for helping the vaping device rotate about the spinner assembly for longer periods of time. The spinner assembly can include a rotor and bearing affixed to said rotor such that the vaping device can be rotated about the rotor.

The method for relieving anxiety, stress or nervousness in a person provides for a vaping device for vaporizing a liquid solution comprising a stress-relieving agent deliverable by inhalation to the person. The vaping device comprises a spinner assembly formed integrally therewith. The method comprises the step of actuating the vaping device to deliver vaporized solution to the person as needed. The method comprises the further step of actuating the spinner assembly to relieve anxiety, stress or nervousness as needed, before, after, or between actuation of the vape device. The method can include a reduction in the frequency of actuating the vaping device by actuating the spinner assembly.

The invention is also directed to a novelty device comprising a combination vaping device and spinner assembly. The combination vaping device and spinner assembly includes a heating element, a power source for actuating the heating element, and a reservoir for storing a liquid solution deliverable to the heating element for heating and vaporization. The heating element, the power source, and the reservoir are mounted on a chassis disposed within a housing. The housing forms the body of the vaping device and a spinner assembly is integrated into or integrally provided in the chassis. The spinner assembly is accessible from the housing of the vaping device and is operated with thumb and finger such that the vaping device is rotatable about the spinner assembly.

### BRIEF DESCRIPTION OF THE DRAWINGS

The drawings as provided herein set forth exemplary embodiments of the present invention, the detailed description of which follows hereinbelow. The drawings are not intended to limit the invention as encompassed by the claims appended herewith.
FIG. 1 is a top elevation view of the vaping device in accordance with an embodiment of the present invention.
FIG. 2A is a perspective view of the vaping device of FIG. 1 with a portion of the housing removed.
FIG. 2B is a perspective view of the portion of the housing removed from the vaping device of FIG. 2A.
FIG. 3 is a top elevation view of the vaping device of FIG. 2A with the spinner assembly removed.
FIG. 4 is an exploded, perspective view of the rotating assembly of the vaping device of FIG. 1.
FIG. 5 is an exploded view of the vaping device of FIG. 1.

### DETAILED DESCRIPTION OF THE INVENTION

The subject invention concerns a combination vaping device and spinner, wherein the spinner is integrated into or integral with the body of the vaping device. A vaping device of the invention functions as a conventional vaping device, comprising a hollow body which includes or houses several components, such as:
∘ A battery, used as a power source, providing the energy needed to heat a coil, which in turn vaporizes the liquid solution to be inhaled. A rechargeable lithium-ion battery is commonly used in vaping devices.
∘ A tank, serving as a reservoir to store the liquid solution.
∘ A coil or atomizer, comprising a small wire-wrapped component made of an alloy, such as Kanthal^{®}, stainless steel, or nickel, serving as a heating element. The battery/power source is activated and sends electrical current through the coil, causing it to heat up, and thereby heat the liquid solution to vaporize it. The vaping device of the invention can include the coil or atomizer within or part of the tank, whereby liquid held within the tank is heated and vaporized. Alternatively, the coil or atomizer can be provided as a component which is separate from the tank, and heats and vaporizes the liquid downstream from the tank.
∘ A liquid solution, which can be referred to as E-liquid, vape solution, vape juice or vape liquid, or the like, for vaporization and inhalation, can be provided separately and filled into the tank as needed. Liquid solutions used in conventional vaping devices can comprise a mixture of propylene glycol (PG), vegetable glycerin (VG), nicotine (optional), flavorings, and the like, and are generally only limited by their ability to be vaporized, free of harmful or toxic substances or irritants, and by their acceptable or desirable taste of the user.
∘ A wick, for retaining or transferring the liquid solution in contact with the coil. Saturation of the coil with liquid solution can optimize efficient vaporization of the liquid solution. The wick is preferably a porous material, such as cotton or other absorbent natural material, or can be a synthetic absorbent material.
∘ Optionally, the vaping device of the invention can include an Airflow Control, providing the user the capability to adjust and customize the amount of air that mixes with the vapor. This can affect the density and temperature of the vapor.

The body of the vaping device according to the subject invention can be an elongated housing that can be fashioned in various shapes and sizes. The elongated body can be generally cylindrical (round, circular, or oval in cross-section) or cuboid or rectangular in cross-section, providing outer faces contoured to be curved or rounded, flat, or both, e.g., having curved top and bottom faces and flat side faces. Other shapes, such as conical, or multifaceted (having more than four faces), are also contemplated. The elongate shape of the vaping device can facilitate holding and using the vaping device.

A preferred vaping device has an elongated body providing a first end comprising a mouthpiece for inhaling vaporized liquid solution and a second end containing the power source for engaging heating element and vaporizing the liquid solution. In one embodiment, the power source can be a rechargeable battery. In another embodiment, the second end can include a charging port connected to the rechargeable battery for recharging the battery. The vaping device body is preferably hollow, forming a cavity within the body to receive and hold the components held within the vaping device body.

Materials forming the vaping device body can include plastic, metal, and even exotic materials like wood or ceramic. The body can be customized with different colors, patterns, and decorative components or adornments, such as LED lights. Such vaping devices are commonly cylindrical, or cuboid (having a cross-section which is square or rectangular in shape, forming a top face, bottom face, left face and right face, and first end face and second end face.)

Vaping devices according to the subject invention, are preferably formed as two mating body pieces - a top body piece and a bottom body piece, that can be adjoined together to form the generally hollow body, i.e. a body shell bounding a cavity formed therein. The adjoining top and bottom pieces can be affixed together using an adhesive or glue, or can be configured to matingly adjoin such that the top and bottom pieces fit or "snap" together to form the single body. The cavity formed by the top and bottom body pieces can include partitions or walls configured to provide spaces shaped to receive components of the vaping device that are held within the cavity when fully assembled.

The vaping body according to the subject invention includes an aperture within the body, which extends through the body, either from top to bottom or from one side to the other side. The aperture, which is preferably circular in shape, and having a diameter which is less than the width of the vaping device body where the aperture is positioned, is walled off from the body such that the aperture is not co-extensive with and is not in communication with the cavity formed by the hollow body or body pieces.

Within the aperture, a spinner is provided, which has an outer rim or defining ring which is affixed to the vaping device body to hold the spinner in place. Positionally, the spinner is provided centrally in relation to the body of the vaping device, meaning that the spinner is positioned along the longitudinal midline of the vaping device and approximately midway between, or equidistant from, each of the first and second ends. Exact placement along the midline or midway between the first and second ends is not critical, but the centralized location is preferred for balance and ease of use of the spinner when handling the vaping device body. For example, a vaping device which is approximately 95 mm in length will preferably have the spinner such that the central axis of rotation is 47.5 mm from either end of the vaping device.

Embodiments of the vaping device of the present invention will be described below with reference to the accompanying drawings.

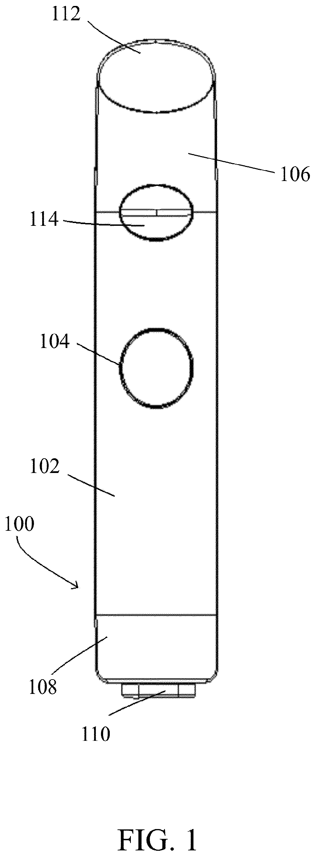

FIG. 1 provides a top elevation view of one embodiment of the present invention. More particularly, FIG. 1 shows a vaping device 100 having an elongated housing 102 with a proximal or inhaling end 106 for oral intake of vaporized liquid solution and a distal or second end 108, the ends opposite one another relative to the lateral axis of the elongated housing 102. Centrally located on the vaping device and approximately equidistant from the inhaling end 106 and the second end 108 is the spinner assembly 104. The spinner assembly 104 provides a center of rotation around which the vaping device 100 is rotatable. It will be appreciated by those skilled in the art that a plurality of housing 102 configurations are possible in multiple embodiments of the invention. For example, the housing 102 can be a singular tubular body. Alternatively, the housing 102 shown in this embodiment includes separable inhaling end 106 and second end 108 portions.

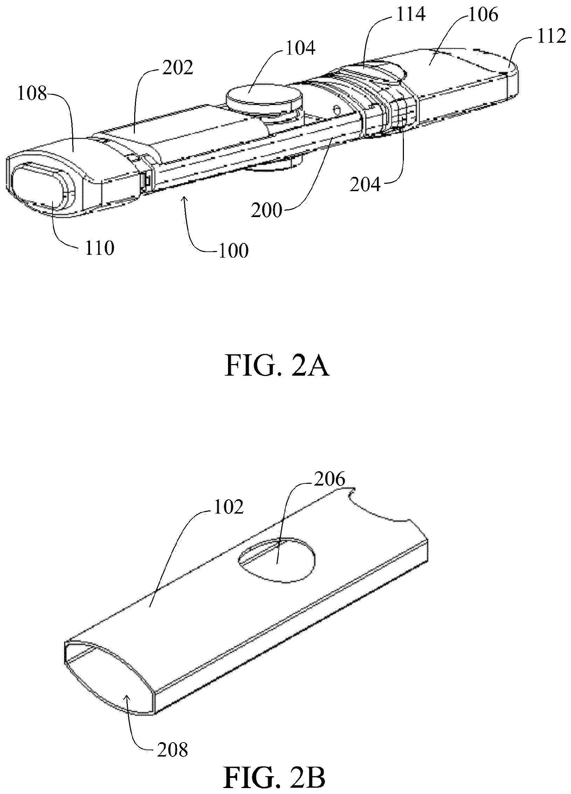

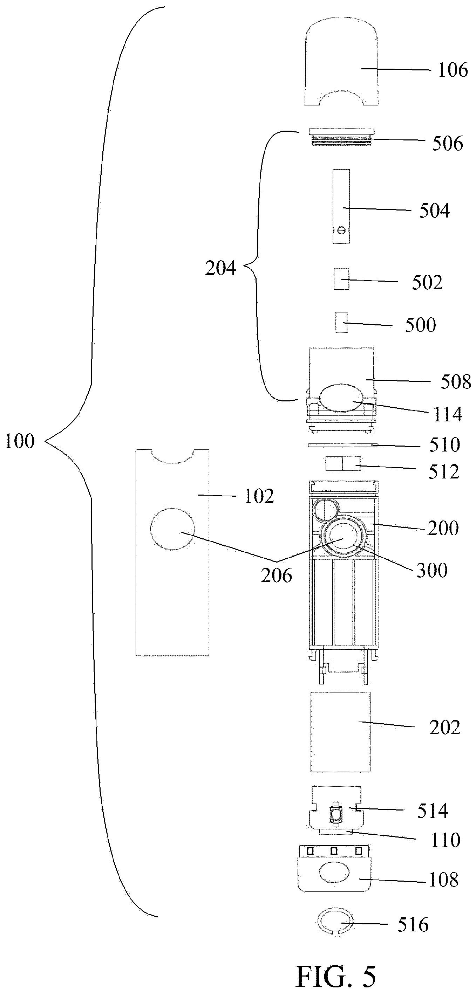

Referring now to FIGS. 2a and 2b, which illustrate the vaping device 100 with a portion of the housing 102 removed and displayed separately. The housing 102 is shown defining a chamber 208 formed to receive and cover the chassis 200. The chassis 200 provides structural support for the housing 102 by strengthening and stiffening it to external forces as well as the internal centrifugal forces caused by rotation of the vaping device 100 about the spinner assembly 104. The chassis 200 is preferably similar in width to the housing 102, such that the lateral surface of the chassis 200 abuts the internal lateral surface of the housing 102 providing lateral support to the housing 102. Additionally, the chassis 200 provides a support structure on which the power source 202, reservoir 204, spinner assembly 104, and heating element 500 (shown in FIG. 5) can be affixed. The reservoir 204 is configured to hold a bulk amount of liquid solution for vaporizing by the heating element 500. The power source 202 is communicatively coupled to the heating element 500 and actuates the heating element 500 on and off. In a preferred embodiment, the power source 202 is rechargeable and in communication with the charging port 110 for recharging when needed. The rechargeable power source 202 is preferably a rechargeable battery such as a lithium-ion battery and the like.

Turning back to FIG. 1, the embodiment of the vaping device 100 shown includes a second end 108 with a dorsally disposed charging port 110. The proximal portion of the inhaling end 106 preferably tapers, forming a mouthpiece 112. In one embodiment, the inhaling end 106 portion of the housing 102 is disposable and replaceable for increased hygiene between vaping sessions. In another embodiment, the mouthpiece portion 112 portion of the inhaling end 106 is disposable and replaceable.

The reservoir 204 (shown in FIG. 2a) can include a viewing window 114 accessible from the distal portion of the inhaling end 106. The viewing window 114 provides users with a visual reading of the amount of solution remaining within the reservoir 204. It would be understood that the viewing window 114 can be provided in a variety of shapes and configurations and is not limited to a window, but can include other forms of visualizing the quantity of solution remaining such as by digital display and the like.

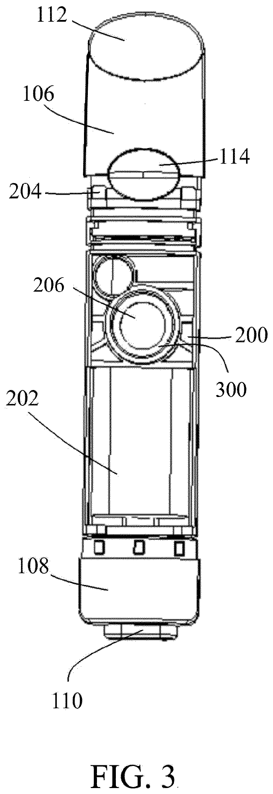

The spinner assembly 104 is accessible through the housing 102 via the aperture 206 (shown in FIG. 2B) which extends vertically through the housing 102. The aperture 206 aligns with the spinner assembly 104 such that a user can grip the spinner assembly with thumb and finger to rotate the vaping device 100. It will be appreciated from FIG. 3 that the aperture 206 also extends through the chassis 200 such that the spinner assembly 104 can advantageously be detached and replaced when needed. The spinner assembly 104 is preferably coupled by a retaining ring 300 affixed to the chassis 200. The retaining ring 300 provides the spinner assembly 104 with the movement necessary to rotate the vaping device 100 while maintaining its position within the housing 102.

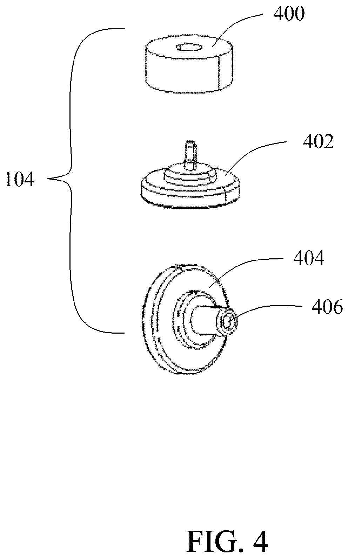

Referring now to FIG. 4, which illustrates an exploded view of the spinner assembly 104 comprising the bearing 400, spinner top cap 402, and spinner base 404. The protruding portion of the spinner top cap 402 is matingly engaged to the spinner base 404 via the spinner base opening 406. The bearing 400 is disposed between the spinner top cap 402 and the spinner base 404 such that the bearing 400 can rotate independently from the spinner top cap 402 and spinner base 404. The retaining ring 300 (shown in FIG. 3) is rotatably affixed to the bearing 400 such that the spinner cap 402 and spinner base 404 rotate independently from the rest of the vaping device 100.

Turning back to FIG. 1, it would be understood by those skilled in the art that the position of the spinner assembly 104 on the vaping device 100 is not critical to the invention as long as it is accessible and can be operated to rotate the vaping device 100 about the spinner assembly 104. The spinner assembly 104 is therefore not limited to being centrally located and can be disposed on any other part of the vaping device 100 that benefits balance and ease of use of the spinner assembly 104 when handling the vaping device 100. In some embodiments, the spinner assembly 104 can be located off-center depending on the center of mass, balance, and shape of the vaping device 100.

FIG. 5 provides an exploded view of the vaping device according to one embodiment of the invention. In this embodiment, counterweight 512 is shown disposed between the chassis 200 and the reservoir 204. The counterweight 512 provides increased balance and ease of use of the vaping device 100 by adjusting its center of mass. In this embodiment, the counterweight 512 is positioned proximally to the spinner assembly 104 such that the center of mass is displaced away from the relatively heavy power source 202.

The reservoir 204 is shown having a reservoir body 508 and a reservoir cover 506 that threadedly engages the reservoir body 508. The distal end of the reservoir body 508 couples to and is supported by the chassis 200. The bulk amount of liquid solution contained within the reservoir 204 is sealed from the chassis 200 by the O-ring 510 which is disposed between the chassis 200 and the reservoir body 508.

The heating element 500 is shown disposed within the reservoir body 508 along with the wick 502 and central tube 504. The wick 502 is configured to absorb, retain, and deliver liquid solution from the reservoir 204 to the heating element 500. The central tube 504 is coupled to the reservoir cover 506 on one end and to the heating element 500 on the other end such that the central tube 504 provides a pathway by which the liquid solution that is vaporized by the heating element 500 can be channeled through the reservoir cover 506 and into the inhaling end 106. In one embodiment, the density and temperature of the vaporized liquid solution can be adjusted through an Airflow Control. The airflow control can be disposed in, and operated from the housing 102 allowing the user to control the amount of air drawn into the vaping device with each inhalation.

The power source 202 can be actuated by sensors configured to detect inhalation by the user. The sensors can be placed on portions of the vaping device 100 where user produced airflow can be detected. For example, sensors can be placed in the inhaling end 106 of the device or in the central tube 504. Alternatively, an actuator button in operational communication with the power source 202 and heating element 500 can be disposed in the housing 102. The power source 202 shown in this embodiment is communicatively coupled to the Printed Circuit Board Assembly (PCBA) 514. The PCBA 514 is attached dorsally to the chassis 200 and comprises a charging port 110 by which the power source 202 can be recharged. The charging port 110 can include a charging port cover 516 for protecting the charging port 110 from water, particles, and other sources of external damage.

In addition to a device, the present invention is also directed to a method for relieving anxiety, stress or nervousness in a person using the structures described herein. For example, a novel method for relieving anxiety, stress or nervousness in a person includes providing the vaping device 100 shown in FIG. 1 with a bulk amount of liquid solution.

The method comprises the steps of
- actuating the vaping device to deliver vaporized solution to the person as needed, and
- actuating the spinner assembly to relieve anxiety, stress, or nervousness as needed, before, after, or between actuations of the vape device.

Actuation of the spinner assembly 104 can help reduce stress and further reduce the frequency of actuating the vaping device. Additionally, rotation of the vaping device 100 advantageously mixes the bulk amount of liquid solution contained within the reservoir 204 providing for improved taste and concentration of vapor.

The method can comprise the additional step of actuating the spinner assembly to mix the liquid solution as needed, before or between actuations of the vape device 100. In addition, the method can comprise the step of wherein actuating the spinner assembly can further reduce the frequency of actuating the vaping device.

The foregoing description of the invention is illustrative only and is not intended to limit the scope of the invention to the precise terms set forth. Further, although the invention has been described in detail with reference to certain illustrative embodiments, variations and modifications exist within the scope and spirit of the invention as described and defined in the following claims.

The above disclosure and example generally describe the present invention and is provided for purposes of illustration and is not intended to limit the scope of the invention. The invention described herein may be practiced in the absence of any element or elements, limitation or limitations which is not specifically disclosed herein. Thus, for example, in each instance herein, any of the terms "comprising," "consisting essentially of," and "consisting of" may be replaced with either of the other two terms. The terms and expressions are used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the claims.

## Claims

1. A vaping device comprising:
a chassis having a heating element, a power source for actuating the heating element, and a reservoir for storing a liquid solution deliverable to said heating element for being heated and vaporized;
a spinner assembly centrally disposed within the chassis such that the vaping device is rotatable about the spinner assembly;
a housing having an inhaling end and a second end, said housing defining a chamber for receiving the chassis and an aperture aligning with the spinner assembly such that the spinner assembly can be accessed with thumb and finger.

2. The vaping device of claim 1, wherein the spinner assembly comprises a rotor and a bearing affixed to said rotor.

3. The vaping device of claim 1, wherein the spinner assembly is affixed to the chassis by a positioning ring.

4. The vaping device of claim 1, wherein the power source is a rechargeable power source.

5. The vaping device of claim 4, wherein the chassis comprises a charging port in communication with the rechargeable power source.

6. The vaping device of claim 5, wherein the charging port is disposed at the second end of the housing.

7. The vaping device of claim 1, wherein the inhaling end comprises a mouthpiece.

8. The vaping device of claim 7, wherein the mouthpiece is disposable.

9. The vaping device of claim 1, wherein the chassis further comprises a wick for delivering liquid solution from the reservoir to the heating element.

10. The vaping device of claim 1, wherein the vaping device further comprises an airflow control.

11. The vaping device of claim 1, wherein the vaping device further comprises a counterweight disposed within the chamber.

12. The vaping device of claim 1, wherein the device further comprises an actuator button disposed in the housing and in operational communication with the heating element for turning the heating element on and off.

13. A method for relieving anxiety, stress or nervousness in a person in need thereof, said method comprising:
providing a vaping device for vaporizing a liquid solution comprising a stress-relieving agent deliverable by inhalation to the person, wherein the vaping device further comprises a spinner assembly integrally formed therein;
actuating the vaping device to deliver vaporized solution to the person as needed, and
actuating the spinner assembly to relieve anxiety, stress, or nervousness as needed, before, after, or between actuations of the vape device.

14. The method of claim 13, wherein actuating the spinner assembly can further reduce the frequency of actuating the vaping device.

15. A novelty device comprising:
a combination vaping device and spinner assembly, wherein the vaping device comprises a heating element, a power source for actuating the heating element, and a reservoir for storing a liquid solution deliverable to said heating element for being heated and vaporized, wherein the heating element, power source, and reservoir are mounted on a chassis disposed within a housing forming a body of the vaping device, and a spinner assembly integrally provided in the chassis and accessible from the body such that the vaping device is rotatable about the spinner assembly.
